# EUROPEAN PATENT SPECIFICATION

(11) **EP 2 777 639 B1**
(45) Date of publication and mention of the grant of the patent: **22.04.2020**
(21) Application number: 14158492.0
(22) Date of filing: 10.03.2014
(51) Int. Cl.: A61F 2/86, A61F 2/91, A61B 90/00, A61L 31/18

(54) **Improved radiopaque marker for vascular devices**
Verbesserter strahlungsundurchlässiger Marker für Gefäßvorrichtungen
Marqueur radio-opaque amélioré pour dispositifs vasculaires

(30) Priority: 11.03.2013 US 201313793474
(43) Date of publication of application: 17.09.2014
(73) Proprietor: DePuy Synthes Products, LLC, Raynham, MA 02767 (US)
(72) Inventor: Lorenzo, Juan A., Raynham, Massachusetts 02767 (US); Girnary, Hussein, Raynham, Massachusetts 02767 (US)
(74) Representative: Carpmaels & Ransford LLP

(56) References cited:
- EP-A1- 2 545 887
- WO-A1-01/72240
- DE-A1-102011 015 995
- US-A1- 2008 243 227

## Description

### BACKGROUND OF THE INVENTION

### 1. Field of the Invention

The invention relates to implants and other devices insertable within body vessels and more particularly to mechanisms for enhancing tracking and location of stents and other vascular devices, especially expandable implants.

### 2. Description of the Related Art

DE102011015995 relates to a medical device that is insertable into a hollow body organ, comprising a compressible and expandable braid form filaments, in particular of wires arranged in rows cross-overs, said cross-over itself filaments have different spiral directions. It is characterized in that at least one connecting element in at least one row of said crossovers, which engages around at least two filaments having different spiral directions, each of the crossovers coming in the connecting element are brought together and after each of the connecting element are further led to a further crossing.

Vascular disorders and defects such as aneurysms, embolisms, and other arterio-venous malformations are especially difficult to treat when located near critical tissues or where ready access to a malformation is not available. Both difficulty factors apply especially to cranial aneurysms. Due to the sensitive brain tissue surrounding cranial blood vessels and the restricted access, it is very challenging and often risky to surgically treat defects of the cranial vasculature.

Alternative treatments include vascular occlusion devices such as stents and embolic coils deployed using delivery catheters having a distal end positioned at an occlusion or aneurysm. Several types of stent delivery systems are disclosed in U.S. Patent Publication No. 2005/0049670 by Jones et al., for example. It is critical to accurately position stents and other vascular devices. Surgeons often seek to confirm correct placement of vascular devices using one or more imaging systems.

Typically, a stent-like vascular reconstruction device is first guided beneath the aneurysm to be treated using a delivery catheter. One commercially available reconstruction product is the CODMAN ENTERPRISE® Vascular Reconstruction Device and System as described, for example, in a Navigate Tough Anatomy brochure Copyright 2009 by Codman & Shurtleff, Inc., 325 Paramount Drive, Raynham, Massachusetts. The CODMAN ENTERPRISE® stent device is carried by a central delivery wire and initially held in place on the delivery wire in a collapsed state by a sheath-type introducer. Typically, a delivery catheter such as a PROWLER® SELECT® Plus microcatheter, also commercially available from Codman & Shurtleff and as disclosed by Gore et al. in U.S. Patent No. 5,662,622, for example, is first positioned intravascularly with its distal tip slightly beyond the neck of the aneurysm. The tapered distal tip of the introducer is mated with the proximal hub of the delivery catheter, and the delivery wire is then advanced through the delivery catheter.

The CODMAN ENTERPRISE® stent device has a highly flexible, self-expanding closed cell design with a number of coils of radiopaque wire to serve as markers at each flared end of the device, similar to the stent illustrated in the published patent application by Jones et al., cited above. Manufacture of such markers is relatively time-consuming and expensive due to the small size of the stent and the need to wrap the radiopaque wire multiple times around struts on the stent, which is especially difficult within closed cells of the stent.

Stent-like, generally non-deployable devices are also utilized to treat disorders arising from embolisms and atherosclerosis. An embolism is the sudden obstruction of a blood vessel by blood clots, cholesterol-containing plaques, masses of bacteria and other debris. A blood clot which obstructs a blood vessel is also referred to as a thrombus. If the embolic obstruction occurs in the brain, it can cause a sudden loss of neurological function referred to as a stroke, in particular an acute ischemic stroke.

A number of devices for treating embolic strokes and atherosclerotic deposits are described for example in U.S. Patent No. 5,972,019 by Engelson et al. with one or more radio-opaque coils of wires "to provide a measure of radio-opacity to the distal tip" and thereby assist tracking of the device during use. A method of monitoring positioning of polymeric stents is disclosed by Sabaria in U.S. Patent Publication No. 2009/0076594. Other, more recent neurological devices include the Micrus Revasc™ of Codman & Shurtleff, Inc., the Solitaire™ device of Microtherapeutics, Inc. d/b/a ev3 Neurovascular, and the Trevo™ and Merci Retreiver™ devices from Concentric Medical.

It is therefore desirable to have an improved device marking system which assists locating and/or positioning vascular devices during and/or after insertion to treat a vascular malformation.

### SUMMARY OF THE INVENTION

An object of the present invention is to provide a radiopaque marker capable of being placed quickly and reliably over a strut or other elongated member of a vascular device.

Another object of the present invention is to provide a stent or other vascular device with highly visible radiopaque markers positioned as desired.

This invention may be expressed as a combination of at least one marker with a stent insertable within vasculature of a patient. The stent includes a plurality of interconnected struts. The marker includes an elongated body formed of a biocompatible radiopaque material that enhances locating the marker when using at least one imaging technique, the body having a first end, a second end, an inner surface, an outer surface, and at least two opposing edges extending between the first and second ends and establishing a boundary between the inner surface and the outer surface. The inner surface of the body defines a passageway extending between the first and second ends. In a first condition, the body defines a gap between the at least two opposing edges, the gap enabling unobstructed communication of the passageway with the outer surface of the body and enabling insertion of a strut of the plurality of interconnected struts into the passageway. In a second condition, the opposing edges are brought into substantial abutment with each other to obstruct the gap to substantially prevent communication of the passageway with the outer surface of the body and to prevent unintended removal of the marker from the stent, thereby securing the marker to the stent such that a single strut of the plurality of interconnected struts securely carries the marker.

In certain embodiments, the marker is positioned between two projections which restrict longitudinal movement of the marker. At least one of the projections is one of the supports for the strut in some embodiments. In a number of embodiments, the device is a stent having a compressed condition during insertion through vasculature and an expanded condition after it is positioned at a desired location. In one embodiment, the strut carrying the marker is part of a closed, deformable cell of the stent.

This invention may be further expressed as a method of enhancing locatability of a stent within vasculature of a patient, including selecting a stent having a plurality of interconnected struts, and a marker having an elongated body formed of a biocompatible radiopaque material that enhances locating the marker when using at least one imaging technique. The body has a first end, a second end, an inner surface, an outer surface, and at least two opposing edges extending between the first and second ends and establishing a boundary between the inner surface and the outer surface. The inner surface of the body defines a passageway extending between the first and second ends. The body initially defines a gap between the at least two opposing edges, the gap enabling unobstructed communication of the passageway with the outer surface of the body. The method further includes inserting a single strut of the plurality of interconnected struts into the passageway, and bringing the opposing edges into substantial abutment with each other to obstruct the gap to substantially prevent communication of the passageway with the outer surface of the body and to prevent unintended removal of the marker from the stent, thereby securing the marker to the stent such that the single strut securely carries the marker.

### BRIEF DESCRIPTION OF THE DRAWINGS

In what follows, preferred embodiments of the invention are explained in more detail with reference to the drawings, in which:
FIG. 1 is a schematic perspective view of a stent illustrating several types of inventive radiopaque markers;
FIG. 2 is a detail view of a free end portion of a strut showing a first inventive marker illustrated in FIG. 1;
FIG. 3 is an exploded view of the strut and radiopaque marker of FIG. 2;
FIG. 4 is a detail view of a strut forming a closed, expandable cell for the stent of FIG. 1 with a marker according to the present invention;
FIG. 5 is an exploded view of the strut and radiopaque marker of FIG. 4;
FIG. 6 is a front elevational view of a strut having a pair of projections according to an aspect of the present invention;
FIG. 7 is a front elevational view of the strut of FIG. 6 with a marker according to the present invention;
FIG. 8 is an exploded view of another marker according to the present invention and another strut from the stent of FIG. 1;
FIG. 9 shows the marker of FIG. 8 placed over a portion of the strut of FIG. 8;
FIG. 10 shows the marker of FIG. 9 after it is deformed to bring opposing edges substantially into abutment to establish a closed seam; and
FIG. 11 shows the marker of FIG. 10 after several welds have been applied.

### DETAILED DESCRIPTION OF THE PRESENTLY PREFERRED EMBODIMENTS

This invention may be accomplished by a combination of a marker and a vascular stent, where the terms "vascular" and "vasculature" are utilized in their broadest meaning to include any duct or tube network in a human or other animal. A marker according to the present invention includes an elongated body formed of a biocompatible radiopaque material that enhances locating the marker when using at least one imaging technique. The body has a first end, a second end, an inner surface, an outer surface, and at least two opposing edges extending between the first and second ends and establishing a boundary between the inner surface and the outer surface. The inner surface of the body defines a passageway extending between the first and second ends. In a first condition, the body defines a gap between the at least two opposing edges, the gap enabling unobstructed communication of the passageway with the outer surface of the body. In a second condition, the opposing edges are brought into substantial abutment with each other to obstruct the gap to substantially prevent communication of the passageway with the outer surface of the body.

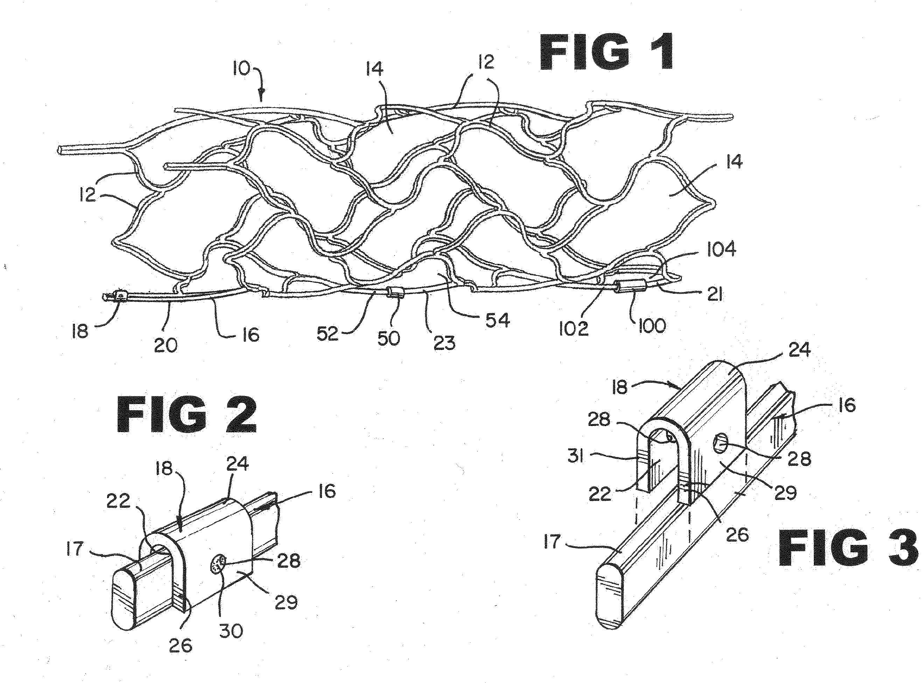

A stent 10, FIG. 1, is implantable in the vasculature of a patient, preferably within a cranial aneurysm. Stent 10 is a substantially tubular vascular device in this construction that is formed of a plurality of interconnected struts 12 manufactured by laser cutting, water jet cutting, etching, or other known methods. The struts 12 define a plurality of cells 14 which are expandable and/or deformable to allow the stent 10 to move between a small-diameter "compressed" or "delivery" condition, typically during manufacture and delivery into a patient, and a large-diameter "expanded" or "deployed" condition, as shown in FIG. 1, when a selected treatment site within vasculature is reached. Stents with markers according to the present invention are self-expanding in some constructions, balloon-expandable in other constructions, or a combination thereof.

One strut 16 is illustrated with a radiopaque marker 18 according to the parent application of the first-named inventor of the present application, published as U.S. Patent Publication No. 2008/0243227, and referred to hereinafter as "Lorenzo 2007" for its inventorship and filing date of March 30, 2007. The strut 16 is shown as a free end portion at a proximal end 20 of the stent 10, but it will be appreciated that radiopaque markers according to the present invention may be incorporated into any strut of a vascular device. A desired number of markers can be utilized, such as two, three or four markers on each end of a vascular implant. As described in more detail below, stent 10 further includes an inventive marker 50 at a central region 23 and an inventive marker 100 at a distal end 21 for illustrative purposes.

The strut 16 and marker 18 are shown in greater detail in FIGS. 2 and 3. The marker 18 has an inner surface 22 adapted for engagement with the strut 16. Preferably, the curvature of inner surface 22 is similar to that of the portion of the strut to be engaged, such as generally "U" or "C"-shaped in this construction, that is, arcuate in cross-section, to mate with the curved surface 17 of the strut 16.

The marker 18 includes an outer surface 24 which is spaced away from the inner surface 22 by a thickness 26, which is uniform in some constructions and non-uniform in other constructions. A portion of the outer surface 24 may be selected or configured for one or more functions, such as engaging with a delivery or deployment device.

The marker 18 further includes at least one through-hole 28 according to the invention of Lorenzo 2007. Marker 18 has two through-holes 28 in this construction, as shown in FIG. 3 before a weld 30, FIG. 2, is applied. Portions 29 and 31 of marker 18 can be crimped against strut 16 to further secure marker 18 to strut 16.

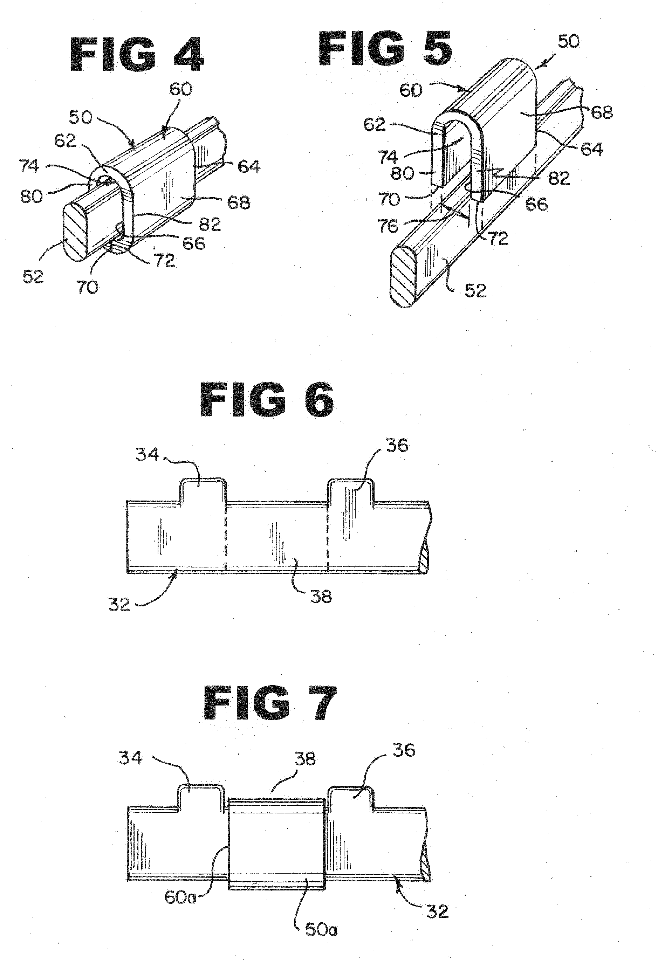

Markers 50 and 100, FIG. 1, according to the present invention preferably are sized to more fully surround a selected strut 52 and 102 of closed cells 54 and 104, respectively, and do not require a through-hole. As shown in FIGS. 4 and 5, marker 50 includes an elongated body 60 formed of a biocompatible radiopaque material that enhances locating the marker when using at least one imaging technique. The body 60 has a first end 62, a second end 64, an inner surface 66, an outer surface 68, and at least two opposing edges 70 and 72 extending between the first and second ends 62, 64 and establishing a boundary between the inner surface 66 and the outer surface 68. The inner surface 66 of the body 60 defines a passageway 74 extending between the first and second ends 62, 64.

In a first condition shown in FIG. 5, the body 60 defines a gap 76 between the at least two opposing edges 70, 72. The gap 76 enables unobstructed communication of the passageway 74 with the outer surface 68 of the body such that strut 52 can be inserted through gap 76 and into passageway 74 to achieve the combination shown in FIG. 4.

In a second condition, FIG. 4, the gap 76 is obstructed to substantially prevent communication of the passageway 74 with the outer surface 68 of the body 60. In this construction, gap 76 is occluded by bring opposing edges 70 and 72 into substantial abutment to establish a closed seam, such as by deforming body 60, that is, by squeezing, clamping or crimping leg portions 80 and 82 together, preferably to clamp marker 50 to strut 52. In other constructions, at least a portion of gap 76 is occluded by a weld or other bridging or attachment technique.

FIG. 6 is a front elevational view of a strut 32 having a pair of projections 34 and 36 according to an aspect of the present invention. The projections may take any number of shapes, including semi-circular, squared, triangular, or an annular configuration extending along a perimeter or other portion of the strut 32, or smaller post- or nipple-like structure extending radially away from the strut 32. Further, the shapes of projections 34 and 36 may differ from each other. At least one projection is integrally formed with strut 32 in some constructions and, in other constructions, is applied as a separate manufacturing step. In yet other constructions, one or both of the projections 32 and 34 are transversely extending support members connected to strut 32, such as other struts defining a closed cell together with strut 32.

Projections 34 and 36 define a receiving region or surface 38 between them, shown in dashed lines in FIG. 6. A marker 50a according to the present invention is shown in FIG. 7 secured to strut 32 with the inner surface 66a lying substantially against receiving surface 38 in a second condition similar to that of marker 50 in FIG. 4. Preferably, the distance between the projections is selected to be substantially equal to the length of marker 50a, FIG. 7, such that marker 50a is held against possible longitudinal movement along the strut 32 and is held at a known geometric position within stent 10.

As illustrated in FIG. 7, the height of each projection is greater than the thickness of the marker. However, the height of the projection or projections may be substantially equal to the thickness of the marker, or even less than the thickness of the marker, provided that the projection or projections, when utilized, are at least configured to abut one or both ends of the marker to minimize or prevent longitudinal movement of the marker.

Projections are especially useful when marker 50a is secured to strut 32 only by clamping or crimping. Projections also serve to accurately position markers at exact locations on struts of a vascular device.

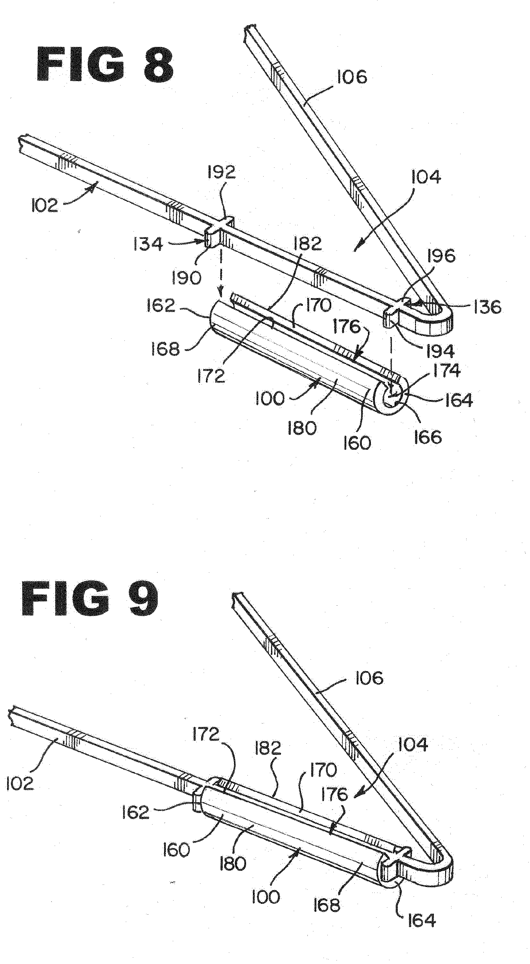

FIG. 8 is an exploded view of another marker 100 according to the present invention and another strut 102 from the stent of FIG. 1. Marker 100 includes an elongated body 160 formed of a biocompatible radiopaque material that enhances locating the marker when using at least one imaging technique. The body 160 has a first end 162, a second end 164, an inner surface 166, an outer surface 168, and at least two opposing edges 170 and 172 extending between the first and second ends 162, 164 and establishing a boundary between the inner surface 166 and the outer surface 168. The inner surface 166 of the body 160 defines a passageway 174 extending between the first and second ends 162, 164.

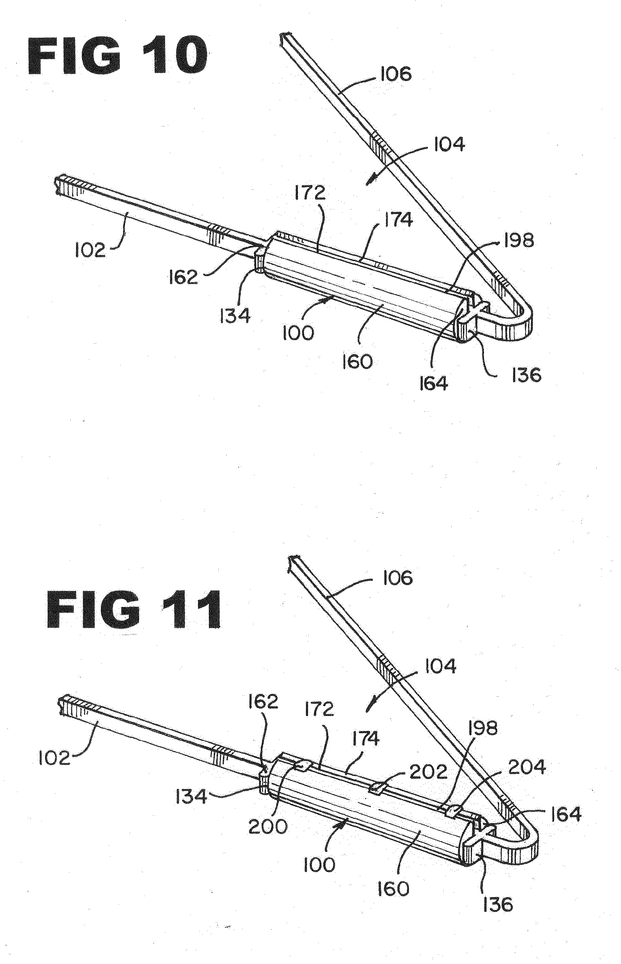

In one construction suitable for treating cranial aneurysms, body 160 is substantially cylindrical in at least one of a first condition and a second condition, is approximately 0.5mm to 2.0 mm in length, more preferably approximately 1.0 mm in length, and is preferably formed from a hypotube having an outer diameter of approximately 0.2 mm (0.008 inch), and inner diameter of 0.1mm (0.004 inch). One suitable source of such a hypotube, formed of a platinum alloy or tantalum, is Johnson Matthey Medical Components (see "www.jmmedical.com"). A gap 176 is formed in one construction by eliminating a section of the hypotube by laser cutting, leaving body 160 as substantially cylindrical in a first condition. In another construction, a thin cut is made to form opposing edges 170 and 172, and then body 160 is deformed to open a gap 176; body 160 is returned to a cylindrical shape in a second condition as described below for FIGS. 10 and 11. Markers according to the present invention, also referred to as marker bands, can have different cross sections to optimize imaging profile and/or maximize radiopacity, including cross sections which are circular, square, or oval.

In a first condition shown in FIGS. 8 and 9, the body 160 defines a gap 176 between the at least two opposing edges 170, 172. The gap 176 enables unobstructed communication of the passageway 174 with the outer surface 168 of the body such that strut 102 can be inserted through gap 176 and into passageway 174 to achieve the combination shown in FIG. 9, with marker 100 placed over a portion of the strut of FIG. 8 between projections 134 and 136.

In a second condition, FIG. 10, the gap 176 is obstructed to substantially prevent communication of the passageway 174 with the outer surface 168 of the body 160. In this construction, gap 176 is occluded by bring opposing edges 170 and 172 into substantial abutment to establish a closed seam 198, such as by deforming body 160, that is, by squeezing, clamping or crimping leg portions 180 and 182 together, preferably to clamp marker 100 to strut 102. In other constructions, at least a portion of gap 176 is occluded by a weld or other bridging or attachment technique. FIG. 10 shows the marker of FIG. 9 after it is deformed to bring opposing edges substantially into abutment.

Projections 134 and 136 include opposing pairs of stops 190, 192 and 194, 196 as shown most clearly in FIG. 8. The spacing between pairs of stops or other projections can be altered at different geometric locations within and about stent 10 or other vascular device to accommodate markers of different diameters and lengths. Further, a support such as another strut 106 can replace one or both projections, such as strut 106 obviating the need for projection 136 for a marker having a longitudinal length greater than that of marker 100.

FIG. 11 shows the marker of FIG. 10 after several optional welds 200, 202 and 204 have been applied. Markers according to the present invention can be soldered, glued or welded to further secure the marker to the stent or other vascular device.

Thus, while there have been shown, described, and pointed out fundamental novel features of the invention as applied to a preferred embodiment thereof, it will be understood that various omissions, substitutions, and changes in the form and details of the devices illustrated, and in their operation, may be made by those skilled in the art without departing from the scope of the invention. For example, it is expressly intended that all combinations of those elements and/or steps that perform substantially the same function, in substantially the same way, to achieve the same results be within the scope of the invention. Substitutions of elements from one described embodiment to another are also fully intended and contemplated. It is also to be understood that the drawings are not necessarily drawn to scale, but that they are merely conceptual in nature. It is the intention, therefore, to be limited only as indicated by the scope of the claims appended hereto.

## Claims

1. A combination of at least one marker (50, 100) with a stent (10) insertable within vasculature of a patient, comprising:
the stent (10) including a plurality of interconnected struts (12, 50, 102);
the marker (50, 100) including an elongated body (60, 160) formed of a biocompatible radiopaque material that enhances locating the marker when using at least one imaging technique, the body having a first end (68, 268), a second end (64, 164), an inner surface (66, 166), an outer surface (68, 168), and at least two opposing edges (70, 72, 170, 172) extending between the first and second ends and establishing a boundary between the inner surface and the outer surface;
the inner surface of the body defining a passageway (74, 174) extending between the first and second ends;
in a first condition, the body defines a gap (76, 176) between the at least two opposing edges, the gap enabling unobstructed communication of the passageway with the outer surface of the body and enabling insertion of a strut of the plurality of interconnected struts into the passageway; and
in a second condition, the opposing edges are brought into substantial abutment with each other to obstruct the gap to substantially prevent communication of the passageway with the outer surface of the body and to prevent unintended removal of the marker from the stent, thereby securing the marker to the stent such that a single strut of the plurality of interconnected struts securely carries the marker.

2. A method of enhancing locatability of a stent (10) within vasculature of a patient, comprising:
selecting a stent (10) including a plurality of interconnected struts (12, 52, 102);
selecting a marker (50, 100) including an elongated body (60, 160) formed of a biocompatible radiopaque material that enhances locating the marker when using at least one imaging technique, the body having a first end (62, 162), a second end (64, 164), an inner surface (66, 266), an outer surface (68, 168), and at least two opposing edges (70, 72, 170, 172) extending between the first and second ends and establishing a boundary between the inner surface and the outer surface, the inner surface of the body defining a passageway (74, 174) extending between the first and second ends, the body initially defining a gap (76, 176) between the at least two opposing edges, the gap enabling unobstructed communication of the passageway with the outer surface of the body;
inserting a single strut of the plurality of interconnected struts into the passageway; and
bringing the opposing edges into substantial abutment with each other to obstruct the gap to substantially prevent communication of the passageway with the outer surface of the body and to prevent unintended removal of the marker from the stent, thereby securing the marker to the stent such that the single strut securely carries the marker.

3. The combination of claim 1 or the method of claim 2 wherein the body is formed of a malleable radiopaque material.

4. The combination of claim 1 or the method of claim 2 wherein the body is substantially cylindrical in at least one of the first and second conditions.

5. The combination of claim 1 or the method of claim 2 wherein the gap is obstructed at least in part by at least one weld (200, 202).

6. The combination of claim 1 or the method of claim 2 wherein the marker is positioned between two projections (34, 36) which restrict longitudinal movement of the marker.

7. The combination of claim 1 or the method of claim 2 wherein the stent has a compressed condition during insertion through vasculature and an expanded condition after it is positioned at a desired location.

8. The combination or the method of claim 7 wherein the single strut carrying the marker is part of a closed, deformable cell (14) of the stent.

## Patentansprüche

1. Kombination von mindestens einem Marker (50, 100) mit einem in die Blutgefäße eines Patienten einführbaren Stent (10), umfassend:
einen Stent (10), der eine Vielzahl von miteinander verbundenen Streben (12, 50, 102) aufweist,
den Marker (50, 100), der einen länglichen Körper (60, 160) aufweist, der aus einem biokompatiblen radiopaken Material gebildet ist, das die Lokalisierung des Markers verbessert, wenn mindestens eine Abbildungstechnik verwendet wird, wobei der Körper ein erstes Ende (68, 268), ein zweites Ende (64, 164), eine Innenfläche (66, 166), eine Außenfläche (68, 168) und mindestens zwei gegenüberliegende Ränder (70, 72, 170, 172), die sich zwischen dem ersten und dem zweiten Ende erstrecken und eine Grenze zwischen der Innenfläche und der Außenfläche bilden, wobei die Innenfläche des Körpers einen sich zwischen dem ersten und dem zweiten Ende erstreckenden Durchgang (74, 174) definiert,
wobei der Körper in einem ersten Zustand einen Spalt (76, 176) zwischen den mindestens zwei sich gegenüberliegenden Rändern definiert, wobei der Spalt eine ungehinderte Verbindung des Durchgangs mit der Außenfläche des Körpers ermöglicht und das Einführen einer Strebe der Vielzahl von miteinander verbundenen Streben in den Durchgang ermöglicht, und
die gegenüberliegenden Ränder in einem zweiten Zustand zum Sperren des Spalts im Wesentlichen zum Anliegen aneinander gebracht werden, um eine Verbindung des Durchgangs mit der Außenfläche des Körpers im Wesentlichen zu verhindern und ein unbeabsichtigtes Entfernen des Markers aus dem Stent zu verhindern, wodurch der Marker so an dem Stent befestigt ist, dass eine einzelne Strebe der Vielzahl von miteinander verbundenen Streben den Marker sicher trägt.

2. Verfahren zur Verbesserung der Lokalisierbarkeit eines Stents (10) in den Blutgefäßen eines Patienten, umfassend:
Wählen eines Stents (10), der eine Vielzahl von miteinander verbundenen Streben (12, 52, 102) aufweist, Wählen eines Markers (50, 100), der einen länglichen Körper (60, 160) aufweist, der aus einem biokompatiblen radiopaken Material gebildet ist, das die Lokalisierung des Markers verbessert, wenn mindestens eine Abbildungstechnik verwendet wird, wobei der Körper ein erstes Ende (62, 162), ein zweites Ende (64, 164), eine Innenfläche (66, 266), eine Außenfläche (68, 168) und mindestens zwei gegenüberliegende Ränder (70, 72, 170, 172), die sich zwischen dem ersten und dem zweiten Ende erstrecken und eine Grenze zwischen der Innenfläche und der Außenfläche bilden, wobei die Innenfläche des Körpers einen sich zwischen dem ersten und dem zweiten Ende erstreckenden Durchgang (74, 174) definiert, wobei der Körper anfänglich einen Spalt (76, 176) zwischen den mindestens zwei sich gegenüberliegenden Rändern definiert, wobei der Spalt eine ungehinderte Verbindung des Durchgangs mit der Außenfläche des Körpers ermöglicht,
Einführen einer einzelnen Strebe der Vielzahl von miteinander verbundenen Streben in den Durchgang und Bringen der gegenüberliegenden Ränder im Wesentlichen zum Anliegen aneinander zum Sperren des Spalts, um eine Verbindung des Durchgangs mit der Außenfläche des Körpers im Wesentlichen zu verhindern und ein unbeabsichtigtes Entfernen des Markers aus dem Stent zu verhindern, wodurch der Marker so an dem Stent befestigt ist, dass die einzelne Strebe den Marker sicher trägt.

3. Kombination nach Anspruch 1 oder Verfahren nach Anspruch 2, wobei der Körper aus einem umformbaren radiopaken Material gebildet ist.

4. Kombination nach Anspruch 1 oder Verfahren nach Anspruch 2, wobei der Körper in dem ersten und/oder zweiten Zustand im Wesentlichen zylindrisch ist.

5. Kombination nach Anspruch 1 oder Verfahren nach Anspruch 2, wobei der Spalt mindestens teilweise durch mindestens eine Schweißnaht (200, 202) gesperrt ist.

6. Kombination nach Anspruch 1 oder Verfahren nach Anspruch 2, wobei der Marker zwischen zwei Vorsprüngen (34, 36) positioniert ist, die eine Längsbewegung des Markers einschränken.

7. Kombination nach Anspruch 1 oder Verfahren nach Anspruch 2, wobei der Stent einen komprimierten Zustand während des Einführens durch Blutgefäße und einen expandierten Zustand nach seiner Positionierung an einem gewünschten Ort hat.

8. Kombination oder Verfahren nach Anspruch 7, wobei die den Marker tragende einzige Strebe Teil einer geschlossenen deformierbaren Zelle (14) des Stents ist.

## Revendications

1. Combinaison d'au moins un marqueur (50, 100) avec une endoprothèse (10) insérable dans le système vasculaire d'un patient, comprenant :
l'endoprothèse (10) comprenant une pluralité d'entretoises interconnectées (12, 50, 102) ;
le marqueur (50, 100) comprenant un corps allongé (60, 160) formé d'un matériau radio-opaque biocompatible qui améliore la localisation du marqueur lorsqu'on utilise au moins une technique d'imagerie, le corps ayant une première extrémité (68, 268), une seconde extrémité (64, 164), une surface intérieure (66, 166), une surface extérieure (68, 168) et au moins deux bords opposés (70, 72, 170, 172) s'étendant entre les première et seconde extrémités et établissant une limite entre la surface intérieure et la surface extérieure ;
la surface intérieure du corps définissant un passage (74, 174) s'étendant entre la première et la seconde extrémité ;
dans un premier état, le corps définit un espace (76, 176) entre les au moins deux bords opposés, l'espace permettant une communication non obstruée du passage avec la surface extérieure du corps et permettant l'insertion d'une entretoise parmi la pluralité d'entretoises interconnectées dans le passage ; et
dans un second état, les bords opposés sont mis sensiblement en butée l'un contre l'autre pour obstruer l'espace afin d'empêcher sensiblement la communication du passage avec la surface extérieure du corps et pour empêcher le retrait involontaire du marqueur de l'endoprothèse, ce qui permet de fixer le marqueur à l'endoprothèse de telle sorte qu'une seule entretoise parmi la pluralité d'entretoises interconnectées porte le marqueur en toute sécurité.

2. Procédé pour améliorer la localisabilité d'une endoprothèse (10) dans le système vasculaire d'un patient, consistant à :
choisir une endoprothèse (10) comprenant une pluralité d'entretoises interconnectées (12, 52, 102) ;
choisir un marqueur (50, 100) comprenant un corps allongé (60, 160) formé d'un matériau radio-opaque biocompatible qui améliore la localisation du marqueur lors de l'utilisation d'au moins une technique d'imagerie, le corps ayant une première extrémité (62, 162), une seconde extrémité (64, 164), une surface intérieure (66, 266), une surface extérieure (68, 168) et au moins deux bords opposés (70, 72, 170, 172) s'étendant entre les première et seconde extrémités et établissant une limite entre la surface intérieure et la surface extérieure, la surface intérieure du corps définissant un passage (74, 174) s'étendant entre les première et seconde extrémités, le corps définissant initialement un espace (76, 176) entre les au moins deux bords opposés, l'espace permettant une communication non obstruée du passage avec la surface extérieure du corps ;
insérer une seule entretoise parmi la pluralité d'entretoises interconnectées dans le passage ; et
mettre les bords opposés en butée l'un contre l'autre pour obstruer l'espace afin d'empêcher sensiblement la communication du passage avec la surface extérieure du corps et pour empêcher le retrait involontaire du marqueur de l'endoprothèse, ce qui permet de fixer le marqueur à l'endoprothèse de telle sorte que ladite seule entretoise porte le marqueur en toute sécurité.

3. Combinaison selon la revendication 1 ou procédé selon la revendication 2, le corps étant formé d'un matériau malléable radio-opaque.

4. Combinaison selon la revendication 1 ou procédé selon la revendication 2, le corps étant sensiblement cylindrique dans au moins l'un des premier et second états.

5. Combinaison selon la revendication 1 ou procédé selon la revendication 2, l'espace étant obstrué au moins en partie par au moins une soudure (200, 202).

6. Combinaison selon la revendication 1 ou procédé selon la revendication 2, le marqueur étant positionné entre deux projections (34, 36) qui limitent le mouvement longitudinal du marqueur.

7. Combinaison selon la revendication 1 ou procédé selon la revendication 2, l'endoprothèse présentant un état comprimé pendant son insertion dans le système vasculaire et un état expansé après avoir été positionnée à un endroit souhaité.

8. Combinaison ou procédé selon la revendication 7, la seule entretoise portant le marqueur faisant partie d'une cellule fermée et déformable (14) de l'endoprothèse.
